(19) Europäisches Patentamt
European Patent Office
Office européen des brevets

(11) Veröffentlichungsnummer: **0 028 780**
**B1**

# (12) EUROPÄISCHE PATENTSCHRIFT

(45) Veröffentlichungstag der Patentschrift: **27.07.83**

(51) Int. Cl.³: **C 07 D 317/72**, A 61 K 7/46

(21) Anmeldenummer: **80106708.3**

(22) Anmeldetag: **31.10.80**

(54) **2-Alkyl-1,4-dioxaspiro(4,n)alkane, deren Herstellung und Verwendung als Riechstoffe, sowie diese enthaltende Riechstoffkompositionen.**

(30) Priorität: **08.11.79 DE 2945049**

(43) Veröffentlichungstag der Anmeldung: **20.05.81 Patentblatt 81/20**

(45) Bekanntmachung des Hinweises auf die Patenterteilung: **27.07.83 Patentblatt 83/30**

(84) Benannte Vertragsstaaten: **CH DE FR GB IT LI NL**

(56) Entgegenhaltungen:
**AT-B-296 299**
**DE-C-936 572**
**US-A-3 022 222**
**US-A-3 491 152**
**US-A-3 919 251**
**US-A-3 919 252**

(73) Patentinhaber: **Henkel Kommanditgesellschaft auf Aktien, Postfach 1100 Henkelstrasse 67, D-4000 Düsseldorf 1 (DE)**

(72) Erfinder: **Schaper, Ulf-Armin, Dr., Nixenstrasse 17, D-4000 Düsseldorf 13 (DE)**
Erfinder **Conrad, Jens, Dr., Dürerweg 15, D-4010 Hilden (DE)**
Erfinder: **Bruns, Klaus, Dr., Notburgaweg 6, D-4150 Krefeld-Traar (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

EP 0 028 780 B1

2-Alkyl-1,4-dioxaspiro(4,n)alkane, deren Herstellung und Verwendung als Riechstoffe sowie diese enthaltende Riechstoffkompositionen

Es wurde gefunden, daß 2-Alkyl-1,4-dioxaspiro(4,n)alkane der allgemeinen Formel

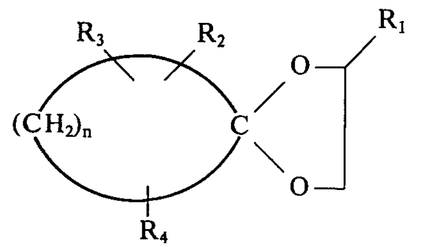

in der $R_1$ einen n-Alkylrest mit 4 – 12 Kohlenstoffatomen, $R_2$, $R_3$ und $R_4$ Wasserstoff oder einen Alkylrest mit 1 – 4 Kohlenstoffatomen und n die Zahlen 4, 5 oder 6 darstellen, wertvolle neue Riechstoffe sind.

Die Herstellung der erfindungsgemäßen neuen Verbindungen erfolgt nach an sich bekannten Syntheseverfahren der organischen Chemie. Als Ausgangsmaterial für die Synthese dienen entsprechende Cyclanone, die mit 1,2-Diolen der Kettenlänge $C_6$ bis $C_{14}$ zu den erfindungsgemäßen Verbindungen gemäß nachfolgendem Reaktionsschema ketalisiert werden. Das bei der Reaktion entstehende Wasser kann durch azeotrope Destillation mit einem geeigneten Lösungsmittel oder Binden mit einem Ameisensäuretriester entfernt werden.

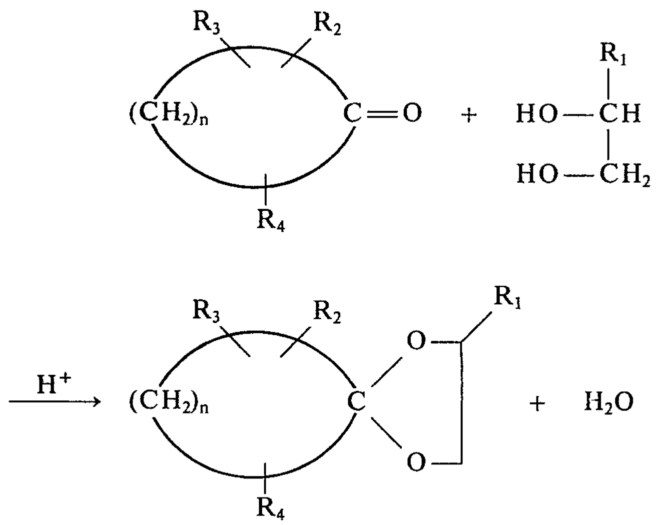


$R_1$, $R_2$, $R_3$, $R_4$ und n kommt hierbei die vorstehend genannte Bedeutung zu. Die Produkte können dabei als isomere Verbindungen vorliegen.

Besondere Bedeutung kommt dem 2-Butyl-1,4-dioxaspiro-(4,4)nonan zu, welches aus Cyclopentanon und 1,2-Hexandiol nach folgendem Reaktionsschema gebildet wird und welches sich durch eine strahlende Jasmon-, Bergamotte-, Linalool-Note auszeichnet.

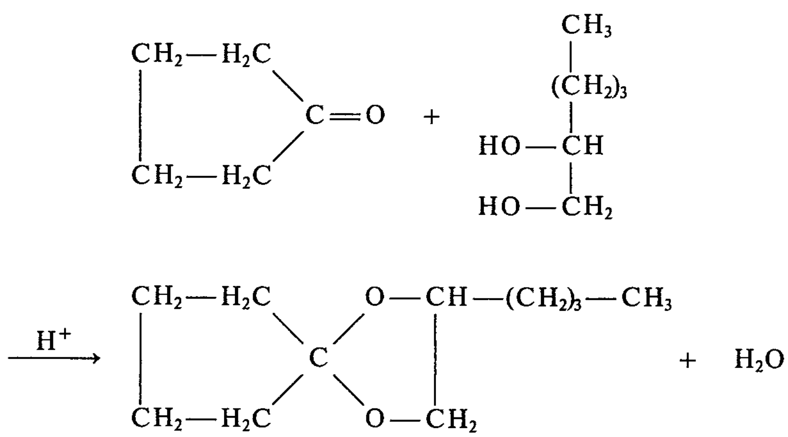


Die erfindungsgemäßen neuen Riechstoffe zeichnen sich durch kräftige, vorwiegend fruchtige und

blumige Geruchsnoten aus, sowie durch eine sehr gute Kombinationsfähigkeit zu neuen, interessanten Kompositionen. Die erfindungsgemäßen neuen Riechstoffe lassen sich mit anderen Riechstoffen in den verschiedensten Mengenverhältnissen mischen. Im allgemeinen wird sich ihr Anteil in den Riechstoffkompositionen in den Mengen von 1 bis 50 Gewichtsprozent, bezogen auf die gesamte Komposition, bewegen. Derartige Kompositionen können zur Parfümierung von kosmetischen Präparaten wie Cremes, Lotionen, Duftwässern, Aerosolen, Toiletteseifen als auch in der Extrait-Parfümerie verwendet werden. Sie können aber auch zur Geruchsverbesserung technischer Produkte wie Wasch- und Reinigungsmittel, Weichspüler, Textilbehandlungsmittel eingesetzt werden. Zur Parfümierung der verschiedenen Produkte werden diesen die Kompositionen im allgemeinen in Konzentrationen von 0,05 bis 2 Gewichtsprozent, bezogen auf das gesamte Produkt, zugesetzt.

Aus US-A 3 022 222 ist es bekannt, daß 8-tert.-Butyl-1,4-dioxaspiro[4,5]decane der allgemeinen Formel

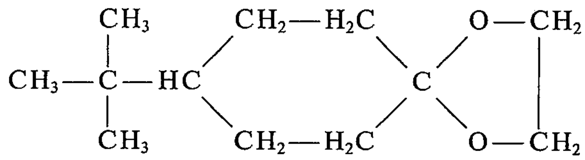

Riechstoffe mit wolliger, erdiger, moschus- und labdanumartiger Geruchsnote sind. Es war jedoch nicht vorhersehbar, daß eine Substitution einer derartigen Verbindung in Position 2 des Dioxolanteils des Ringsystems durch einen n-Alkylrest mit 4–12 Kohlenstoffatomen gemäß der Erfindung zu Riechstoffen mit völlig andersartiger Geruchsnote führen würde.

Die nachfolgenden Beispiele sollen den Gegenstand der Erfindung näher erläutern.

## Beispiele

A) Herstellung von 2-Butyl-1,4-dioxaspiro(4,4)nonan

148 g (1,76 Mol) Cyclopentanon, 189,1 g (1,6 Mol) 1,2-Hexandiol und 5 g p-Toluolsulfonsäure wurden in 1 Liter Cyclohexan unter Auskreisen des Reaktionswassers zum Sieden erhitzt. Nach 1,5 Stunden waren 30 ml Wasser abgetrennt. Das Reaktionsgemisch wurde mit Sodalösung und danach mit Wasser neutral gewaschen, über Natriumsulfat getrocknet und im Vakuum eingeengt. Bei der Destillation im Wasserstrahlvakuum ergab sich eine Ausbeute von 250 g (1,35 Mol, 84% der Theorie) an 2-Butyl-1,4-dioxaspiro(4,4)nonan. Das Produkt besaß eine strahlende Jasmon-, Bergamotte-, Linalool-Geruchsnote und folgende Kennzahlen:

Kp 96° C/19 mbar $n_D^{20}$ 1,4490
NMR ($CCl_4$) $\delta$(ppm): 0,95 m ($CH_3$); 1,4 m ($CH_2$-Kette) 1,7 s ($CH_2$-Ring), 3,3 t (CH), 3,9 m ($CH_2$).

Nach der folgenden Arbeitsvorschrift wurden die nachstehend aufgeführten erfindungsgemäßen neuen Riechstoffe hergestellt:

0,1 Mol entsprechendes Cyclanon, 0,1 Mol entsprechendes 1,2-Alkandiol, 0,1 Mol Orthoameisensäuretriethylester und 0,5 g p-Toluolsulfonsäure wurden 1 Stunde lang bei Raumtemperatur verrührt. Danach wurde langsam ein Gemisch aus Ameisensäureethylester und Ethanol abdestilliert. Der abgekühlte Rückstand wurde in Ether aufgenommen, mit Sodalösung und Wasser neutral gewaschen, über Natriumsulfat getrocknet, eingeengt und im Vakuum destilliert.

B) 2-Hexyl-1,4-dioxaspiro(4,4)nonan
Kp 135° C/20 mbar $n_D^{20}$ 1,4520
Geruch: ähnlich A)

C) 2-Octyl-1,4-dioxaspiro(4,4)nonan
Kp 98° C/0,013 mbar $n_D^{20}$ 1,4546
Geruch: fruchtig, krautig

D) 2-Decyl-1,4-dioxaspiro(4,4)nonan
Kp 95° C/0,013 mbar $n_D^{20}$ 1,4568
Geruch: stärker als C)

E) 2-Butyl-6,6,8-trimethyl-1,4-dioxaspiro(4,4)nonan
im Gemisch mit dem 6,8,8-Trimethyl-Isomeren
Kp 115° C/20 mbar $n_D^{20}$ 1,4464
Geruch: fruchtig

F) 2-Hexyl-6,6,8-trimethyl-1,4-dioxaspiro(4,4)nonan
im Gemisch mit dem 6,8,8-Trimethyl-Isomeren
Kp 72° C/0,013 mbar $n_D^{20}$ 1,4498
Geruch: fruchtig

G) 2-Octyl-6,6,8-trimethyl-1,4-dioxaspiro(4,4)nonan im Gemisch mit dem 6,8,8-Trimethyl-Isomeren
Kp 103° C/0,013 mbar $n_D^{20}$ 1,4520
Geruch: fruchtig, blumig

H) 2-Decyl-6,6,8-trimethyl-1,4-dioxaspiro(4,4)nonan im Gemisch mit dem 6,8,8-Trimethyl-Isomeren
Kp 125° C/0,013 mbar $n_D^{20}$ 1,4542
Geruch: blumig, süß

J) 2-Butyl-1,4-dioxaspiro(4,5)decan
Kp 57° C/0,065 mbar $n_D^{20}$ 1,4553
Geruch: Hyacinthen-, Verotyl-Note, blumig

K) 2-Hexyl-1,4-dioxaspiro(4,5)decan
Kp 76° C/0,013 mbar $n_D^{20}$ 1,4570
Geruch: blumig

L) 2-Butyl-8-tert.butyl-1,4-dioxaspiro(4,5)decan
Kp 91° C/0,013 mbar $n_D^{20}$ 1,4619
Geruch: würzig, fruchtig, scharf

M) 2-Hexal-8-tert.butyl-1,4-dioxaspiro(4,5)decan
Kp 110° C/0,013 mbar $n_D^{20}$ 1,4634
Geruch: Pfeffer-Note, würzig, krautig

N) 2-Butyl-1,4-dioxaspiro(4,6)undecan
Kp 71° C/0,013 mbar $n_D^{20}$ 1,4635
Geruch: fruchtig

O) 2-Hexyl-1,4-dioxaspiro(4,6)undecan
Kp 92° C/0,013 mbar $N_D^{20}$ 1,4682
Geruch: fruchtig

## Beispiel 1

### Jasmin-Komplex

| | |
|---|---|
| 2-Butyl-1,4-dioxaspiro(4,4)nonan | 150 Gewichtsteile |
| Benzylacetat | 200 Gewichtsteile |
| alpha-Hexylzimtaldehyd | 200 Gewichtsteile |
| Linalool | 100 Gewichtsteile |
| Benzylsalicylat | 100 Gewichtsteile |
| Geraniol | 50 Gewichtsteile |
| Terpineol | 50 Gewichtsteile |
| beta-Naphthylmethylketon | 30 Gewichtsteile |
| Ylang-Ylang-Öl | 30 Gewichtsteile |
| Petitgrainöl | 30 Gewichtsteile |
| Zimtblätteröl | 20 Gewichtsteile |
| Aldehyd C 14, 10% in DEP | 20 Gewichtsteile |
| Methylheptincarbonat, 10% in DEP | 10 Gewichtsteile |
| Para-Kresylphenylacetat | 10 Gewichtsteile |
| | 1000 Gewichtsteile |

## Patentansprüche

1. 2-Alkyl-1,4-dioxaspiro(4,n)-alkane der allgemeinen Formel

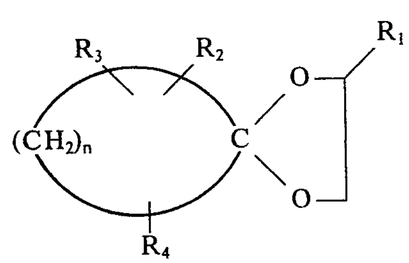

in der $R_1$ einen n-Alkylrest mit 4–12 Kohlenstoffatomen, $R_2$, $R_3$ und $R_4$ Wasserstoff oder einen Alkylrest mit 1–4 Kohlenstoffatomen und n die Zahlen 4, 5 oder 6 darstellen.

2. 2-Butyl-1,4-dioxaspiro(4,4)nonan.

3. Verfahren zur Herstellung der 2-Alkyl-1,4-dioxaspiro(4,n)alkane nach Anspruch 1 und 2, dadurch gekennzeichnet, daß man Cyclanone der allgemeinen Formel

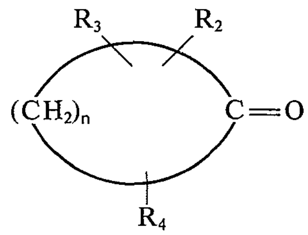

worin $R_2$, $R_3$, $R_4$ und n die in Anspruch 1 angegebene Bedeutung haben, mit 1,2-Diolen der allgemeinen Formel

$$R_1-CHOH-CH_2OH$$

worin $R_1$ ein n-Alkylrest mit 4–12 Kohlenstoffatomen ist, in Gegenwart eines sauren Katalysators ketalisiert.

4. Verwendung der 2-Alkyl-1,4-dioxaspiro(4,n)alkane nach Anspruch 1–2 als Riechstoffe.

5. Riechstoffkompositionen, dadurch gekennzeichnet, daß sie die 2-Alkyl-1,4-dioxaspiro(4,n)alkane nach Anspruch 1–2 in einer Menge von 1–50 Gewichtsprozent, bezogen auf die gesamte Komposition, enthalten.

## Claims

1. 2-Alkyl-1,4-dioxaspiro-(4,n)-alkanes corresponding to the following general formula

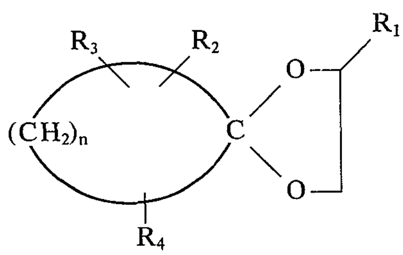

in which $R_1$ is an n-alkyl radical containing from 4 to 12 carbon atoms, $R_2$, $R_3$ and $R_4$ represent hydrogen or a $C_1-C_4$ alkyl radical and n is the number 4, 5 or 6.

2. 2-Butyl-1,4-dioxaspiro-(4,4)-nonane.

3. A process for producing the 2-alkyl-1,4-dioxaspiro-(4,n)-alkanes claimed in Claims 1 and 2 by ketalising cyclanones corresponding to the following general formula

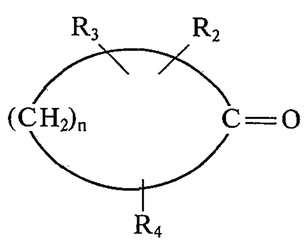

with 1,2-diols corresponding to the general formula $R_1-CHOH-CH_2OH$, in which $R_1$, $R_2$, $R_3$ and $R_4$ and n are as defined above, by synthesis methods generally known in organic chemistry.

4. The use of the 2-alkyl-1,4-dioxaspiro-(4,n)-alkanes claimed in Claims 1 to 3 as perfumes.

5. Perfume compositions, characterised in that they contain the 2-alkyl-1,4-dioxaspiro-(4,n)-alkanes claimed in Claims 1 to 4 in a quantity of from 1 to 50% by weight, based on the composition as a whole.

**Revendications**

1. 2-alkyl-1,4-dioxaspiro(4,n)-alcanes de formule générale

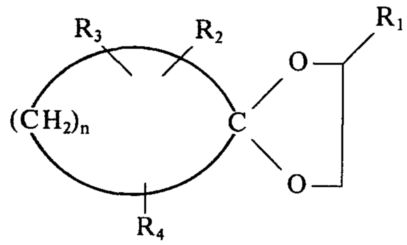

dans laquelle $R_1$ représente un groupe n-alkyle en $C_4-C_{12}$, $R_2$, $R_3$ et $R_4$ représentent l'hydrogène ou un groupe alkyle en $C_1-C_4$ et n représente les nombres 4, 5 ou 6.

2. Le 2-butyl-1,4-dioxaspiro(4,4)nonane.

3. Procédé de préparation des 2-alkyl-1,4-dioxaspiro(4,n)alcanes selon les revendications 1 et 2, par acétalisation de cyclanones de formule générale

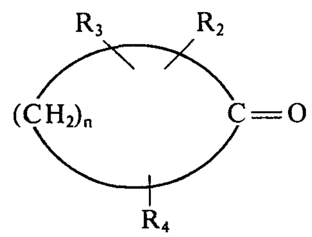

à l'aide de 1,2-diols de formule générale $R_1-CHOH-CH_2OH$, $R_1$, $R_2$, $R_3$, $R_4$ et n ayant les significations indiquées ci-dessus, par des modes opératoires généraux connus de synthèse de la chimie organique.

4. Utilisation des 2-alkyl-1,4-dioxaspiro(4,n)alcanes selon les revendications 1 à 3, en tant que matières aromatiques.

5. Compositions de parfums caractérisées en ce qu'elles contiennent les 2-alkyl-1,4-dioxaspiro(4,n)alcanes selon les revendications 1 à 4 en quantités de 1 à 50% en poids, par rapport à la composition totale.